# FASCICULE DE BREVET EUROPEEN

(11) **EP 0 904 776 B1**
(45) Date de publication et mention de la délivrance du brevet: **26.10.2005**
(21) Numéro de dépôt: 98402117.0
(22) Date de dépôt: 26.08.1998
(51) Int. Cl.: A61K 7/48, A61K 7/42

(54) **COMPOSITIONS PHOTOPROTECTRICES COMPRENANT UN BENZYLIDENE CAMPHRE ET/OU UN DIBENZOYLMETHANE ET/OU UNE TRIAZINE ET UN TARTRATE DE DIALKYLE, UTILISATIONS EN COSMETIQUE**
SONNENSCHUTZMITTEL ENTHALTEND BENZYLIDENKAMPFER UND/ODER DIBENZOYLMETHANE UND/ODER TRIAZINDERIVATE UND DIALKYLTARTRAT, SOWIE IHRE KOSMETISCHE VERWENDUNG
SUN SCREENING PREPARATIONS COMPRISING A BENZYLIDENE CAMPHER AND/OR A DIBENZOYLMETHANE AND/OR TRIAZIN DERIVATIVES AND A DIALKYLTARTRATE, IT'S COSMETIC USE

(30) Priorité: 09.09.1997 FR 9711173
(43) Date de publication de la demande: 31.03.1999
(73) Titulaire: L'OREAL, 75008 Paris (FR)
(72) Inventeur: Josso, Martin, 75005 Paris (FR); Refregier, Jean-Louis, 78700 Conflans-Sainte-Honorine (FR); Hansenne, Isabelle, Westfield, New Jersey 07090 (US)
(74) Mandataire: Miszputen, Laurent

(56) Documents cités:
- EP-A- 0 635 260
- WO-A-95/05154
- WO-A-96/40047
- US-A- 5 091 171

## Description

La présente invention concerne de nouvelles compositions cosmétiques et/ou dermatologiques destinées à la protection de la peau et/ou des cheveux contre les rayonnements UV, en particulier le rayonnement solaire. Plus précisément, elle concerne de nouvelles compositions cosmétiques et/ou dermatologiques comprenant au moins un filtre UV du type dibenzoylméthane et/ou un filtre UV du type dérivé du benzylidéne camphre et/ou d'au moins un filtre UV du type dérivé de triazine présentant un facteur de protection solaire amélioré et comprenant, dans un support cosmétiquement et/ou dermatologiquement acceptable au moins un tartrate de dialkyle, linéaire ou ramifié.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est-à-dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogéne avec le filtre UV au temps nécessaire pour atteindre le seuil érythématogène sans filtre UV).

Or, à la suite d'importantes recherches menées dans le domaine de la photoprotection évoqué ci-dessus, la Demanderesse a maintenant découvert, de façon inattendue et surprenante, qu'il est possible d'obtenir des compositions antisolaires ayant des indices de protection améliorés en associant un tartrate de di-alkyle en C₁-C₁₅ et plus particulièrement en C₁₂-C₁₅ à un système photoprotecteur constitué d'au moins un filtre UV du type dérivé du benzylidéne camphre et/ou un filtre UV du type du type dibenzoylméthane et/ou d'au moins un filtre UV du type dérivé de triazine, en tous cas supérieurs à ceux qui peuvent être obtenus avec un tel système photoprotecteur seul.

Cette propriété est d'autant plus surprenante que l'on connaît dans l'art antérieur notamment dans la demande WO96/40047 des compositions cosmétiques ou pharmaceutiques contenant des esters d'alpha-hydroxyacide notamment le tartrate de diéthyle en présence de filtres UV classiques et plus particulièrement, dans la demande EP-A-635 260 des lipogels antisolaires utilisant des tartrates de dialkyle en C₁₂-C₁₃ et des filtres UV du type cinnamique ou benzophénone. On connaît également des compositions photoprotectrices à base de 2,4,6-tris[p-(2'-éthylhexyl-1'-oxycarbonyl)-anilino]-1,3,5-triazine (vendue notamment sous la dénomination commerciale de "UVINUL T150" par la Société BASF) dans lesquelles on utilise en plus des dialkylesters d'acide carboxylique α,β-dihydroxyle uniquement pour solubiliser ladite triazine ou pour améliorer la solubilisation de ce filtre UV particulier.

La Demanderesse a constaté de façon inattendue que l'indice de protection n'était pas amélioré en utilisant un tartrate de dialkyle en association avec un système photoprotecteur selon l'art antérieur ne contenant pas ceux de l'invention et étant constitué d'autres types de filtres UV classiques tels que des dérivés cinnamiques ou benzophénone.

Cette découverte est à la base de la présente invention.

Ainsi, conformément à un premier objet de la présente invention, il est proposé de nouvelles compositions, plus particulièrement destinées à la protection de la peau et/ou des cheveux contre le rayonnement ultraviolet, caractérisées par le fait qu'elles comprennent, dans un support cosmétiquement acceptable, au moins :
(a) un système photoprotecteur capable de filtrer le rayonnement UV, constitué d'au moins un filtre UV du type dérivé du benzylidéne camphre et/ou d'au moins un filtre UV du type dérivé du dibenzoylméthane et/ou d'au moins un filtre UV du type dérivé de triazine ;
(b) un tartrate de dialkyle, linéaire ou ramifié ayant de préférence de 1 à 16 atomes de carbone ;
ladite composition ne contenant pas le 2,4,6-tris[p-(2'-éthylhexyl-1'-oxycarbonyl)anilino]-1,3,5-triazine.

Selon l'invention, on entend désigner de manière générale par système photoprotecteur capable de filtrer le rayonnement UV, tout composé ou toute association de composés qui, par des mécanismes connus en soi d'absorption et/ou de réflexion et/ou diffusion du rayonnement UV-A et/ou UV-B, permet d'empêcher, ou du moins limiter, la mise en contact dudit rayonnement avec une surface (peau, cheveux,) sur laquelle ce ou ces composés ont été appliqués. En d'autres termes, ces composés peuvent être des filtres organiques photoprotecteurs absorbeurs d'UV ou des (nano)pigments minéraux diffuseurs et/ou réflecteurs d'UV, ainsi que leurs mélanges.

Un autre objet encore de la présente invention réside dans l'utilisation d'un tartrate de dialkyle pour la fabrication d'une composition cosmétique ou dermatologique destinées à la protection de la peau et/ou des cheveux contre le rayonnement ultraviolet, du type comprenant un système photoprotecteur capable de filtrer le rayonnement UV, constitué d'au moins un filtre UV du type dérivé du benzylidéne camphre et/ou d'au moins un filtre UV du type du type dibenzoylméthane et/ou d'au moins un filtre UV du type dérivé de triazine, pour augmenter le facteur de protection solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.
D'autres caractéristiques, aspects et avantages de la présente invention apparaîtront à la lecture de la description détaillée qui va suivre.

On peut citer comme tartrates de dialkyle, les composés linéaires dont les radicaux alkyles sont en C₁-C₄, tels que les tartrates de diméthyle, diéthyle ou dipropyle. On utilisera de préférence le tartrate de diéthyle.

Des tartrates de di-alkyle conformes à la présente invention particulièrement préférés répondent à la structure suivante : 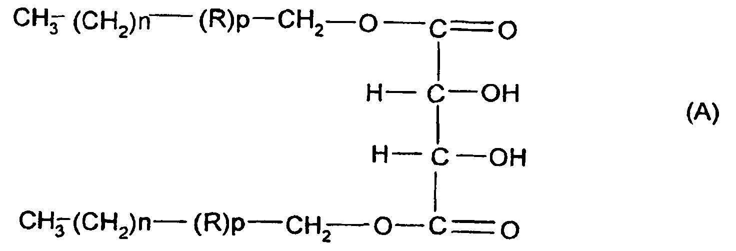 dans laquelle R désigne un groupe de formule : 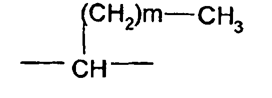 p est 0 ou 1 ;
n vaut 12 ou 13 quand p est égal à 0 et
m + n vaut 8 ou 9 quand p est égal à 1.

On utilisera plus particulièrement les tartrates de di-alkyle linéaire en C₁₂-C₁₃ tels que ceux vendus sous le nom COSMACOL ETI par la Société ENICHEM AUGUSTA INDUSTRIALE ainsi que les tartrates de di-alkyle linéaire en C₁₄-C₁₅ tels que ceux vendus sous le nom COSMACOL ETL par la même société.

Les tartrates de di-alkyle sont utilisés dans les compositions conformes à l'invention à des concentrations allant de préférence de 0,1 à 10% en poids par rapport au poids total de la composition et plus particulièrement de 0,2 à 5% en poids.

Les dérivés du dibenzoylméthane visés par la présente invention sont des produits déjà bien connus en soi et décrits notamment dans les documents FR-A-2326405, FR-A-2440933 et EP-A-0114607, documents dont les enseignements sont, pour ce qui touche à la définition même de ces produits, totalement inclus à titre de références dans la présente description.

Parmi les dérivés du dibenzoylméthane plus particulièrement visés par la présente invention, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- 4-tert-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane.

Parmi les dérivés du dibenzoylméthane mentionnés ci-dessus, on préfère tout particulièrement, selon la présente invention, mettre en oeuvre le 4-tert-butyl-4'-méthoxydibenzoylméthane, notamment celui proposé à la vente sous la dénomination commerciale de "PARSOL 1789" par la Société Givaudan, ce filtre répondant à la formule développée (I) suivante :

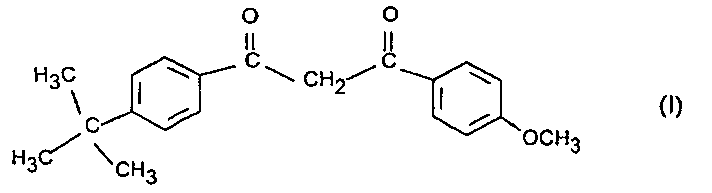

Un autre dérivé du dibenzoylméthane préféré selon la présente invention est le 4-isopropyldibenzoylméthane, filtre vendu sous la dénomination de "EU SOLEX 8020" par la Société Merck, et répondant à la formule développée (II) suivante :

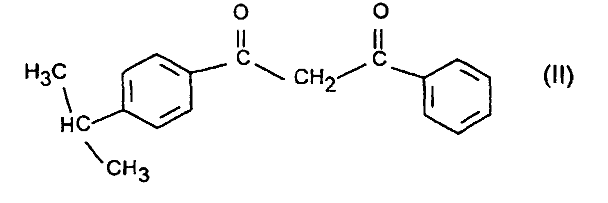

Les dérivés du benzylidène camphre visés par la présente invention sont des produits déjà bien connus en soi. On peut citer à titre d'exemple, les composés suivants :
- le 3-benzylidène camphre ;
- le 4-méthyl-benzylidène camphre ;
- le méthylsulfate de 3(4'-triméthyl-ammoniumbenzylidène)-camphre ;
- l'acide benzylidéne camphre sulfonique et ses sels
- le 3 (3'-sulfo-4'-méthyl benzylidéne) camphre ;
- le polyacrylamido-méthyl benzylidène camphre ;

On peut citer également les diorganopolysiloxanes à fonction benzylidène camphre tels que décrits dans le brevet EP-B-0355777 ainsi que diorganosiloxanes à chaînes courtes, linéaires ou cycliques ou les triorganosilanes à fonction benzylidène camphre tels que décrits dans le brevet EP-B-0712855 ; ces documents étant, pour ce qui touche à la définition même de ces produits, totalement inclus à titre de références dans la présente description.

On peut citer aussi l'acide benzène 1,4-di(3-méthylidéne-10-camphosulfonique) et ses différents sels décrits notamment dans les demandes de brevets FR-A-2528420 et FR-A-2639347, sont des filtres déjà connus en soi (filtres dits à large bande) capables en effet d'absorber les rayons ultraviolets de longueur d'ondes comprises entre 280 nm et 400 nm, avec des maxima d'absorption compris entre 320 nm et 400 nm, en particulier aux alentours de 345 nm. Ces filtres répondent à la formule générale (III) suivante : 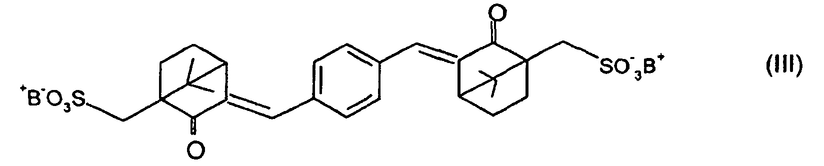 dans laquelle B désigne un atome d'hydrogène, un métal alcalin ou encore un radical NH(R)₃+ dans lequel les radicaux R, qui peuvent être identiques ou différents, désignent un atome d'hydrogène, un radical alkyle ou hydroxyalkyle en C₁-C₄ ou encore un groupement Mⁿ⁺/n, Mⁿ⁺ désignant un cation métallique polyvalent dans lequel n est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺. Il est bien entendu que les composés de formule (III) ci-dessus peuvent donner lieu à l'isomère "cis-trans" autour d'une ou plusieurs double(s) liaison(s) et que tous les isomères rentrent dans le cadre de la présente invention.

On utilisera tout particulièrement le 4-méthylbenzylidène camphre qui est un filtre liposoluble connu en soi, absorbant dans l'UV-B et vendu notamment sous la dénomination commerciale « EUSOLEX 6300 » par la société MERCK. ou de "PARSOL 5000" par la Société GIVAUDAN. Ce produit répond à la formule (IV) suivante :

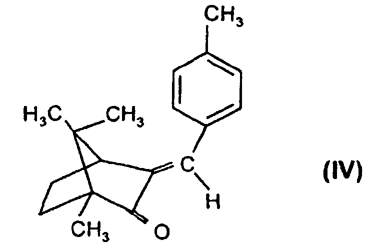

Comme dérivé de triazine, on utilisera plus particulièrement la 2,4,6-tris[p-(2'-éthylhexyl-1'-oxycarbonyl)anilino]-1,3,5-triazine. Ce filtre est connu en soi, actif dans l'UV-B et se présente sous une forme solide, qui est vendue notamment sous la dénomination commerciale de "UVINUL T150" par la Société BASF. Ce produit répond à la formule (V) suivante : 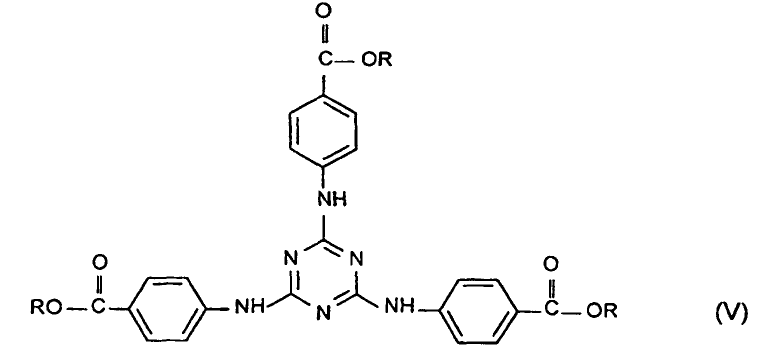 dans laquelle R désigne un radical 2-éthyl hexyle.

Comme dérivés de triazine, on pourra également utiliser ceux décrits dans la demande de brevet FR-A-2744721 répondant à la formule (VI) suivante : 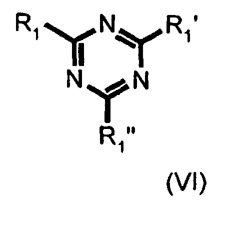 dans laquelle :
- R₁, R₁' et R₁", qui peuvent être identiques ou différents, sont choisis parmi les radicaux monovalents de formules (1) ou (2) suivantes : 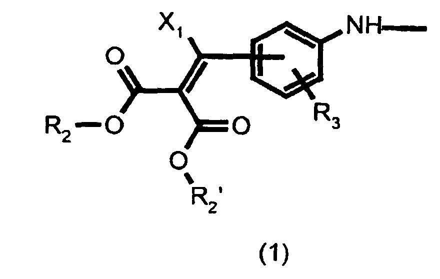 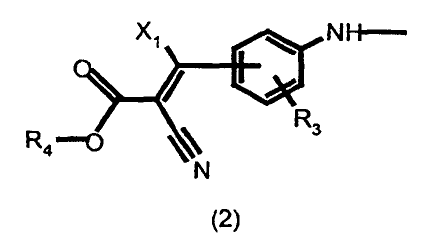
dans lesquelles :
- R₂ et R₂', qui peuvent être identiques ou différents, désignent un radical alkyle en C₁-C₃ linéaire ou ramifié,
- R₃ désigne un atome d'hydrogène, un radical alkyle en C₁-C₄ linéaire ou ramifié ou un radical alkoxy en C₁-C₄,
- R₄ désigne un radical alkyle en C₁-C₁₂ linéaire ou ramifié,
- X₁ représente un atome d'hydrogène ou un radical phényle éventuellement substitué par un halogène ou par un radical alkyle en C₁-C₄ ou par un radical alcoxy en C₁-C₄.

Parmi les composés de formule (VI), on peut citer plus particulièrement les composés suivants :
- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris(a-cyano-4-aminocinnamate d'éthyle)-s-triazine.

On peut également utiliser d'autres dérivés de triazine tels que ceux décrits dans les demandes de brevet EP-A-0517104, EP-A-0570838, EP-A-0796851 ; ces documents étant, pour ce qui touche à la définition même de ces produits, totalement inclus à titre de références dans la présente description.

Les systèmes photoprotecteurs selon l'invention peuvent contenir en plus soit un ou plusieurs filtres organiques additionnels absorbeurs d'UV différents des dérivés du benzylidène camphre, des dérivés du dibenzoylméthane et des dérivés de triazine, soit un ou plusieurs pigments et/ou nanopigments minéraux, soit encore des mélanges entre ceux-ci .

Les filtres UV organiques additionnels utilisables dans les compositions antisolaires conformes à l'invention sont des filtres solaires classiques, actifs dans l'UV-A et/ou l'UV-B, hydrophiles ou lipophiles. A titre d'exemples, ces filtres peuvent être choisis, seuls ou en mélange, parmi l'acide 2-phényl benzimidazole 5-sulfonique et ses sels, les dérivés cinnamiques, les dérivés salicyliques, les dérivés de la benzophénone, les dérivés de β,β-diphénylacrylate, les dérivés de l'acide p-aminobenzoïque, l'anthranilate de menthyle, les polymères filtres et silicones filtres décrits dans la demande WO-93-04665. D'autres exemples de filtres organiques sont donnés dans la demande de brevet EP-A-0487404.

Le système photoprotecteur selon l'invention est généralement présent dans les compositions selon l'invention à une teneur allant de 0,1 % à 30 %, de préférence de 0,5 à 15 %, en poids, par rapport au poids total de la composition.

Une deuxième catégorie d'agents photoprotecteurs additionnels pouvant être utilisés dans les compositions selon l'invention est celle des pigments. De préférence, on met en oeuvre des nanopigments (taille moyenne des particules primaires : généralement entre 5 nm et 100 nm, de préférence entre 10 et 50 nm) minéraux d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflexion et/ou diffusion) du rayonnement UV. Des agents d'enrobage classiques sont par ailleurs l'alumine et/ou le stéarate d'aluminium ou encore les silicones. De tels nanopigments d'oxydes métalliques, enrobés ou non enrobés, sont en particulier décrits dans les demandes de brevets EP-A-0518772 et EP-A-0518773.

Le ou les (nano)pigments minéraux peuvent être présents dans les compositions selon l'invention à une teneur comprise entre 0,1 % et 30 %, de préférence de 0,5 % à 10 %, en poids, par rapport au poids total de la composition.

Les autres constituants pouvant rentrer dans la formulation des compositions visées par l'invention, en particulier les huiles, les composés cireux, les actifs cosmétiques et/ou dermatologiques sont ceux qui sont classiquement utilisés dans le domaine cosmétique et/ou dermatologique.

Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis, de jojoba) ; synthétiques comme le perhydrosqualène, les alcools, les acides ou les esters gras (palmitate d'octyle, lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Les compositions conformes à l'invention peuvent également contenir des agents anti radicaux libres, des antioxydants, des vitamines comme les vitamines E et C, des α-hydroxyacides.

Les compositions selon l'invention peuvent également contenir des agents de bronzage et/ou de brunissage artificiels de la peau (agents autobronzants), tels que par exemple de la dihydroxyacétone (DHA).

Les compositions visées par la présente invention peuvent aussi contenir divers ingrédients classiquement utilisés dans le domaine cosmétique, dermatologique ou dermopharmaceutique comme les matières colorantes, les solvants (eau, alcools,...), les conservateurs, les parfums, les actifs hydratants, des agents pulvérulents, des agents bactéricides et/ou des absorbeurs d'odeur.

Bien entendu, l'homme de l'art veillera à choisir le ou les éventuels composés complémentaires cités ci-dessus et/ou leurs quantités de manière telle que les propriétés avantageuses attachées intrinsèquement à l'association [système photoprotecteur + tartrate d'alkyle en C₁₂-C₁₅] conforme à l'invention ne soient pas, ou substantiellement pas, altérées par la ou les adjonctions envisagées.

Les compositions concernées par l'invention peuvent être préparées selon les techniques bien connues de l'homme de l'art, en particulier celles destinées à la préparation de dispersions à phase continue aqueuse telles que les émulsions de type huile-dans-eau, les gels et les gels-crèmes.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, de poudre, de bâtonnet solide et éventuellement être conditionnée en aérosol et se présenter sous forme de mousse ou de spray.

A titre indicatif, pour les formulations antisolaires conformes à l'invention qui présentent un support de type dispersion à phase continue aqueuse, comme les émulsions huile-dans-eau et les gels-crèmes, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, par rapport à l'ensemble de la formulation.

De préférence, les compositions conformes à l'invention se présentent sous la forme de gels-crèmes.

Des exemples concrets, mais nullement limitatifs, illustrant l'invention, vont maintenant être donnés.

### EXEMPLE1 :

On a préparé une formulation antisolaire B selon l'invention se présentant sous la forme d'une émulsion de type huile-dans-eau et contenant:

### Phase 1 :

- 4-(ter.-butyl) 4'-méthoxy dibenzoylméthane ("PARSOL 1789") 2,5g
- α-cyano-β, β-diphénylacrylate de 2-éthylhéxyle ("UVINUL N 539") 10g
- Tartrate de di-alkyle en C₁₄-C₁₅ ("COSMACOL ETL") 1g
- Acide stéarylique 0,5g
- Acide stéarique 1g
- Diméthicone 1g
- Benzoate d'alcools en C12-C15 vendu sous la dénomination commerciale « Finsolv TN » par la société Finetex 2g
- Mélange stéarate de glycéryle/stéarate PEG-100 vendu sous le nom ARLACEL 165 par ICI 1,5g
- Triéthanolamine 0,3g
- Conservateurs qs

### Phase 2 :

- Acide polyacrylique vendu sous la dénomination commerciale « Carbopol 980 » par Goodrich 0,2g
- Triéthanolamine 0,2g

### Phase 3 :

- Cétyl phoshate de potassium ("AMPHISOL K" de GIVAUDAN) 1g

### Phase 4 :

- Agents hydratants 15g
- Conservateurs qs

### Phase 5 :

- Cyclométhicone 10g

### Phase 6 :

- Acide benzène 1,4-di(3-méthylidène-10-camphosulfonique) 1g
- Triéthanolamine qs pH7
- Eau purifiée qs 100 g

On a ensuite préparé une formulation antisolaire B' comparative, de même support que formulation B mais ne contenant pas de tartrate de di-alkyle en C₁₄-C₁₅.

Pour chacune des compositions B et B', on a ensuite déterminé le facteur de protection solaire (SPF) qui lui était attaché.

Pour chacune des compositions B et B', on a ensuite déterminé le facteur de protection solaire (SPF) qui lui était attaché. Celui-ci a été déterminé en utilisant la méthode *in vitro* décrite par B.L. DIFFEY et al. dans J. Soc. Cosmet. Chem. 40-127-133 (1989) ; cette méthode consiste à déterminer les facteurs de protection monochromatiques tous les 5 nm dans une gamme de longueurs d'onde de 290 à 400 nm et à calculer à partir de ceux-ci le facteur de protection solaire selon une équation mathématique donnée.

Les résultats (valeur moyenne correspondant à cinq essais) sont regroupés dans le tableau **(I)** ci-dessous :

**Tableau (I):**

| Composition | B (invention) avec tartrate de dialkyle | B' (comparative) sans tartrate de dialkyle |
|---|---|---|
| IP moyen | 37,9 | 26,0 |
| (Ecart type) | (4,0) | (5,6) |

Ces résultats montrent clairement que l'ajout d'un tartrate de di-alkyle en C₁₄-C₁₅ à un système photoprotecteur comprenant au moins un filtre UV du type dérivé du dibenzoylméthane et un filtre UV du type dérivé du benzylidène camphre permet d'augmenter significativement son pouvoir photoprotecteur.

### EXEMPLE 2 :

On a préparé une formulation antisolaire C selon l'invention se présentant sous la forme d'une émulsion de type huile-dans-eau et contenant :

### Phase 1 :

- 4-(ter-butyl) 4'-méthoxy dibenzoylméthane ("PARSOL 1789") 5g
- Acide stéarylique 0,5g
- Acide stéarique 1g
- Diméthicone 1g
- Tartrate de di-alkyle en C₁₂-C₁₃ ("COSMACOL ETI") 1g
- Benzoate d'alcools en C12-C15 vendu sous la dénomination commerciale « Finsolv TN » par la société Finetex 2g
- Mélange stéarate de glycéryle/stéarate PEG-100 vendu sous le nom ARLACEL 165 par ICI 1,5g
- Triéthanolamine 0,3g
- Conservateurs qs

### Phase 2 :

- Acide polyacrylique vendu sous la dénomination commerciale « Carbopol 980 » par Goodrich 0,2g
- Triéthanolamine 0,2g

### Phase 3 :

- Cétyl phosphate de potassium ("AMPHISOL K" de GIVAUDAN) 1g

### Phase 4 :

- Agents hydratants 15g
- Conservateurs qs

### Phase 5 :

- Cyclométhicone 10g

### Phase 6 :

- Triéthanolamine qs pH7
- Eau purifiée qs 100 g

On a ensuite préparé une formulation antisolaire C' comparative, de même support que formulation C mais ne contenant pas de tartrate de di-alkyle en C₁₂-C₁₃.

Pour chacune des compositions C et C', on a ensuite déterminé le facteur de protection solaire (SPF) qui lui était attaché. Celui-ci a été déterminé en utilisant la méthode décrite dans l'exemple **1** .

Les résultats (valeur moyenne correspondant à cinq essais) sont regroupés dans le tableau **(II)** ci-dessous :

**Tableau (II) :**

| Composition | C (invention) avec tartrate de dialkyle | C' (comparative) sans tartrate de dialkyle |
|---|---|---|
| SPF moyen | 7,5 | 3,4 |
| (Ecart type) | (1,1) | (0,4) |

Ces résultats montrent clairement que l'ajout d'un tartrate de di-alkyle en C₁₂-C₁₃ à un système photoprotecteur comprenant un filtre UV du type dérivé du dibenzoylméthane permet d'augmenter significativement son pouvoir photoprotecteur. On obtient des résultats équivalents en utilisant le tartrate de diéthyle (C₂) ou un tartrate de di-alkyle en C₁₂-C₁₃ comme le produit COSMACOL ETI à la place du produit COSMACOL ETL.

### EXEMPLE 3 :

On a préparé une formulation antisolaire D selon l'invention ayant la même composition que celle de l'exemple **2** mais contenant à la place du filtre UV du type dérivé du dibenzoylméthane , 6% en poids de 4-méthylbenzylidène camphre " EUSOLEX 6300 " de Merck" et comme tartrate de dialkyle le produit COSMACOL ETL en C₁₄-C_{15.}

On a ensuite préparé une formulation antisolaire D' comparative, de même support que formulation D mais ne contenant pas de tartrate de di-alkyle.

Pour chacune des compositions D et D', on a ensuite déterminé le facteur de protection solaire (SPF) qui lui était attaché. Celui-ci a été déterminé en utilisant la méthode décrite dans l'exemple **1**.

Les résultats (valeur moyenne correspondant à cinq essais) sont regroupés dans le tableau (III) ci-dessous :

**Tableau (III) :**

| Composition | D (invention) avec tartrate de dialkyle | D' (comparative) sans tartrate de dialkyle |
|---|---|---|
| SPF moyen | 12,4 | 6,5 |
| (Ecart type) | (1,9) | (0,7) |

Ces résultats montrent clairement que l'ajout d'un tartrate de di-alkyle en C₁₄-C₁₅ à un système photoprotecteur constitué d'un filtre UV du type dérivé du benzylidène camphre permet d'augmenter significativement son pouvoir photoprotecteur. On obtient des résultats équivalents en utilisant le tartrate de diéthyle (C₂) ou un tartrate de di-alkyle en C₁₂-C₁₃ comme le produit COSMACOL ETI à la place du produit COSMACOL ETL.

### EXEMPLE 4 :

On a préparé une formulation antisolaire E selon l'invention ayant la même composition que celle de l'exemple 3 mais contenant à la place du filtre UV du type dérivé du benzylidène camphre, 5% en poids de la 2,4,6-tris[p-(2'-éthylhexyl-1'-oxycarbonyl)anilino]-1,3,5-triazine ("UVINUL T 150" de la Société BASF)

On a ensuite préparé une formulation antisolaire E' comparative, de même support que formulation E mais ne contenant pas de tartrate de di-alkyle.

Pour chacune des compositions E et E', on a ensuite déterminé le facteur de protection solaire (SPF) qui lui était attaché. Celui-ci a été déterminé en utilisant la méthode décrite dans l'exemple 1.

Les résultats (valeur moyenne correspondant à cinq essais) sont regroupés dans le tableau **(IV)** ci-dessous :

**Tableau (IV) :**

| Composition | E (invention) avec tartrate de dialkyle | E' (comparative) sans tartrate de dialkyle |
|---|---|---|
| SPF moyen | 8,9 | 7,1 |
| (Ecart type) | (0,2) | (0,7) |

Ces résultats montrent clairement que l'ajout d'un tartrate de di-alkyle en C₁₄-C₁₅ à un système photoprotecteur constitué d'un filtre UV du type dérivé de triazine permet d'augmenter significativement son pouvoir photoprotecteur. On obtient des résultats équivalents en utilisant le tartrate de diéthyle (C₂) ou un tartrate de di-alkyle en C₁₂-C₁₃ comme le produit COSMACOL ETI à la place du produit COSMACOL ETL.

### EXEMPLE 5 (comparatif) :

On a préparé une formulation antisolaire F (ne faisant pas partie de l'invention) se présentant sous la forme d'une émulsion de type huile-dans-eau ayant la même composition que celle de l'exemple **3** mais contenant à la place du filtre UV du type dérivé benzylidène camphre, 9% en poids de p-méthoxycinnamate de 2-éthylhexyle ("PARSOL MCX" de chez Givaudan)

On a ensuite préparé une formulation antisolaire F' comparative, de même support que celui de la formulation F mais ne contenant pas de tartrate de di-alkyle en C₁₄-C₁₅.

Pour chacune des compositions F et F', on a ensuite déterminé le facteur de protection solaire (SPF) qui lui était attaché. Celui-ci a été déterminé en utilisant la méthode décrite dans l'exemple 1.

Les résultats (valeur moyenne correspondant à cinq essais) sont regroupés dans le tableau **(V)** ci-dessous :

**Tableau (V) :**

| Composition | F (invention) avec tartrate de dialkyle | F' (comparative) sans tartrate de dialkyle |
|---|---|---|
| SPF moyen | 12,3 | 11,6 |
| (Ecart type) | (1,1) | (2,4) |

Ces résultats montrent clairement que l'ajout d'un tartrate de di-alkyle en C₁₄-C₁₅ à un système photoprotecteur constitué d'un filtre UV du type dérivé d'acide cinnamique ne permet pas d'augmenter significativement son pouvoir photoprotecteur.

### EXEMPLE 6 (comparatif) :

On a préparé une formulation antisolaire G (ne faisant pas partie de l'invention) se présentant sous la forme d'une émulsion de type huile-dans-eau ayant la même composition que celle de l'exemple **3** mais contenant à la place du filtre UV du type dérivé de benzylidène camphre, 6% en poids de benzophénone-3 ("UVINUL M40" de chez BASF).

On a ensuite préparé une formulation antisolaire G' comparative, de même support que celui de la formulation G mais ne contenant pas de tartrate de di-alkyle en C₁₄-C₁₅.

Pour chacune des compositions G et G', on a ensuite déterminé le facteur de protection solaire (SPF) qui lui était attaché. Celui-ci a été déterminé en utilisant la méthode décrite dans l'exemple **1**.

Les résultats (valeur moyenne correspondant à cinq essais) sont regroupés dans le tableau **(VI)** ci-dessous :

**Tableau (VI) :**

| Composition | G (hors invention) avec tartrate de dialkyle | G' (hors invention) sans tartrate de dialkyle |
|---|---|---|
| SPF moyen | 5,4 | 6,3 |
| (Ecart type) | (1,2) | (1) |

Ces résultats montrent clairement que l'ajout d'un tartrate de di-alkyle en C₁₂-C₁₅ à un système photoprotecteur constitué d'un filtre UV du type benzophénone ne permet pas d'augmenter significativement son pouvoir photoprotecteur.

### EXEMPLE 7 (comparatif) :

On a préparé une formulation antisolaire H (ne faisant pas partie de l'invention) se présentant sous la forme d'une émulsion de type huile-dans-eau ayant la même composition que celle de l'exemple **3** mais contenant à la place du filtre UV du type dérivé de benzylidène camphre, 9% en poids de p-méthoxycinnamate de 2-éthylhexyle ("PARSOL MCX" de chez Givaudan) et 1% en poids de tartrate de diéthyle à la place du COSMACOL ETL.

On a ensuite préparé une formulation antisolaire H' comparative, de même support que celui de la formulation H mais ne contenant pas de tartrate de diéthyle.

Pour chacune des compositions H et H', on a ensuite déterminé le facteur de protection solaire (SPF) qui lui était attaché. Celui-ci a été déterminé en utilisant la méthode décrite dans l'exemple 1.

Les résultats (valeur moyenne correspondant à cinq essais) sont regroupés dans le tableau (VII) ci-dessous :

**Tableau (VIII) :**

| Composition | H (hors invention) avec tartrate de dialkyle | H' (hors invention) sans tartrate de dialkyle |
|---|---|---|
| SPF moyen | 11,6 | 12,5 |
| (Ecart type) | (2, 4) | (0,9) |

Ces résultats montrent clairement que l'ajout du tartrate de diéthyle à un système photoprotecteur constitué d'un filtre UV du type cinnamique ne permet pas d'augmenter son pouvoir photoprotecteur.

## Revendications

1. Composition destinée à la protection de la peau et/ou des cheveux contre le rayonnement ultraviolet, **caractérisée par le fait qu'**elle comprend, dans un support cosmétiquement acceptable, au moins :
(a) un système photoprotecteur capable de filtrer le rayonnement UV, constitué d'au moins un filtre UV du type dérivé du benzylidène camphre et/ou d'au moins un filtre UV du type dérivé du dibenzoylméthane et/ou d'au moins un filtre UV du type dérivé de triazine ;
(b) un tartrate de dialkyle, linéaire ou ramifié ayant de préférence de 1 à 16 atomes de carbone ;
ladite composition ne contenant pas la 2,4,6-tris[p-(2'-éthylhexyl-1'-oxycarbonyl)anilino]-1,3,5-triazine.

2. Composition selon la revendication 1, selon laquelle le tartrate de dialkyle est choisi parmi les tartrates de dialkyle en C₁-C₁₆.

3. Composition selon la revendication 1 ou 2, selon laquelle le tartrate de dialkyle est choisi parmi les tartrates de dialkyle en C₁-C₄

4. Composition selon l'une quelconque des revendications 1 à 3, selon laquelle le tartrate de dialkyle est le tartrate de diéthyle

5. Composition selon la revendication 1 ou 2, selon laquelle le tartrate de dialkyle répond à la structure suivante : 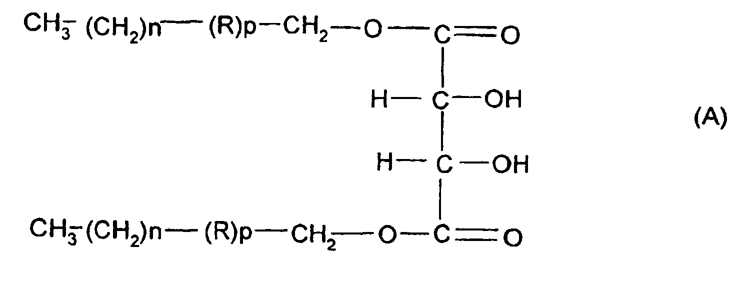 dans laquelle R désigne un groupe de formule : 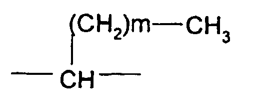 p est 0 ou 1 ;
n vaut 12 ou 13 quand p est égal à 0 et
m + n vaut 8 ou 9 quand p est égal à 1.

6. Composition selon l'une quelcoque des revendications 1 à 6, selon laquelle le tartrate de di-alkyle est utilisé à une concentration allant de 0,1 à 10% en poids par rapport au poids total de la composition et plus particulièrement de 0,2 à 5% en poids.

7. Composition selon l'une quelconque des revendications 1 à 6, selon laquelle le dérivé du dibenzoylméthane est choisi dans le groupe constitué par :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4-tert-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane.

8. Composition selon l'une quelconque des revendications 1 à 7, selon laquelle le dérivé du dibenzoylméthane est le 4-tert-butyl-4'-méthoxydibenzoylméthane.

9. Composition selon l'une quelconque des revendications 1 à 7, selon laquelle le dérivé du dibenzoylméthane est le 4-isopropyldibenzoylméthane.

10. Composition selon l'une quelconque des revendications 1 à 9, selon laquelle le dérivé du benzylidène camphre est choisi dans le groupe constitué par :
- le 3-benzylidène camphre ;
- le 4-méthyl-benzylidène camphre ;
- le méthylsulfate de 3(4'-triméthyl-ammoniumbenzylidène)-camphre ;
- l'acide benzylidène camphre sulfonique et ses sels
- le 3 (3'-sulfo-4'-méthyl benzylidène) camphre ;
- le polyacrylamido- méthyl benzylidène camphre ;

11. Composition selon l'une quelconque des revendications 1 à 9, selon laquelle le dérivé du benzylidène est choisi dans le groupe constitué par :
- les diorganopolysiloxanes à fonction benzylidène camphre ;
- les diorganosiloxanes à chaînes courtes, linéaires ou cycliques ;
- les triorganosilanes à fonction benzylidène camphre.

12. Composition selon l'une quelconque des revendications 1 à 9, selon laquelle le dérivé du benzylidéne correspond à la formule générale (III) suivante : 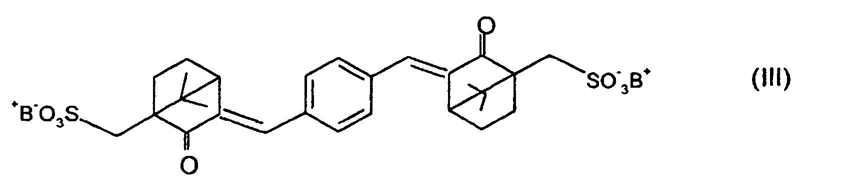 dans laquelle B désigne un atome d'hydrogène, un métal alcalin ou encore un radical NH(R)₃⁺ dans lequel les radicaux R, qui peuvent être identiques ou différents, désignent un atome d'hydrogène, un radical alkyle ou hydroxyalkyle en C₁-C₄ ou encore un groupement Mⁿ⁺/n, Mⁿ⁺ désignant un cation métallique polyvalent dans lequel n est égal à 2 ou 3 ou 4, Mⁿ⁺ désignant de préférence un cation métallique choisi parmi Ca²⁺, Zn²⁺, Mg²⁺, Ba²⁺, Al³⁺ et Zr⁴⁺ ainsi que ses isomères.

13. Composition selon l'une quelconque des revendications 1 à 10, selon laquelle le dérivé du benzylidène camphre est le 4-méthylbenzylidéne camphre.

14. Composition selon l'une quelconque des revendications 1 à 13, selon laquelle le dérivé de triazine répond à la formule (VI) suivante : 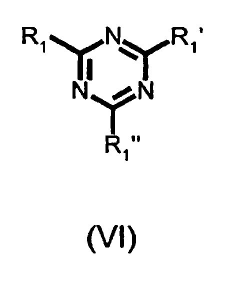 dans laquelle :
- R₁, R₁' et R₁", qui peuvent être identiques ou différents, sont choisis parmi les radicaux monovalents de formules (1) ou (2) suivantes : 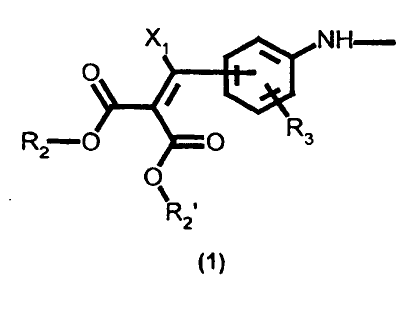 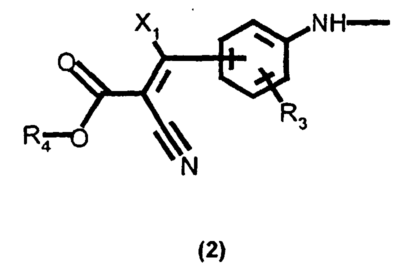
dans lesquelles :
- R₂ et R₂', qui peuvent être identiques ou différents, désignent un radical alkyle en C₁-C₃ linéaire ou ramifié,
- R₃ désigne un atome d'hydrogène, un radical alkyle en C₁-C₄ linéaire ou ramifié ou un radical alkoxy en C₁-C₄,
- R₄ désigne un radical alkyle en C₁-C₁₂ linéaire ou ramifié,
- X₁ représente un atome d'hydrogène ou un radical phényle éventuellement substitué par un halogène ou par un radical alkyle en C₁-C₄ ou par un radical alcoxy en C₁-C₄.

15. Composition selon la revendication 14, selon laquelle le dérivé de triazine est choisi parmi:
- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,
- la 2,4,6-tris(a-cyano-4-aminocinnamate d'éthyle)-s-triazine.

16. Composition selon l'une quelconque des revendications 1 à 15, selon laquelle le système photoprotecteur contient en plus un ou plusieurs filtres organiques additionnels absorbeurs d'UV différents des dérivés du benzylidéne camphre, des dérivés du dibenzoylméthane et des dérivés de triazine, un ou plusieurs pigments et/ou nanopigments minéraux ou des mélanges entre ceux-ci .

17. Composition selon la revendication 16, selon laquelle les filtres UV organiques additionnels sont choisis, seuls ou en mélanges, parmi l'acide 2-phényl benzimidazole 5-sulfonique et ses sels, les dérivés cinnamiques, les dérivés salicyliques, les dérivés de la benzophenone, les dérivés de β,β-diphénylacrylate, les dérivés de l'acide p-aminobenzoïque, l'anthranilate de menthyle, les polymères filtres et silicones filtres.

18. Composition selon l'une quelconque des revendications 1 à 17, selon laquelle le système photoprotecteur est présent dans ladite composition à une concentration allant de 0,1 % à 30 % et de préférence de 0,5 à 15 % , en poids, par rapport au poids total de la composition.

19. Composition selon l'une quelconque des revendications 1 à 18, **caractérisée par le fait que** le système photoprotecteur comprend en outre, à titre d'agents photoprotecteurs complémentaires, des pigments ou des nanopigments d'oxydes métalliques, enrobés ou non, capables de bloquer physiquement, par diffusion et/ou réflection, le rayonnement UV.

20. Composition selon la revendication 19, **caractérisées par le fait que** lesdits pigments ou nanopigments sont choisis parmi les oxydes de titane, de zinc, de fer, de zirconium, de cérium et leurs mélanges, enrobés ou non.

21. Composition selon l'une quelconque des revendications 1 à 20, **caractérisée par le fait qu'**elles comprend en outre au moins un agent de bronzage et/ou de brunissage artificiel de la peau.

22. Composition selon l'une quelconque des revendications 1 à 21, **caractérisée par le fait qu'**elle se présente sous la forme d'une dispersion à phase continue aqueuse.

23. Composition selon la revendication 22, **caractérisée par le fait qu'**elle se présente sous la forme d'un gel-crème.

24. Utilisation d'un tartrate de dialkyle tel que défini dans l'une quelconque des revendication 1 à 3 pour la fabrication d'une composition destinée à la protection de la peau et/ou des cheveux contre le rayonnement ultraviolet, du type comprenant un système photoprotecteur capable de filtrer le rayonnement UV, constitué d'au moins un filtre UV du type dérivé du benzylidène camphre et/ou d'au moins un filtre UV du type du type dibenzoylméthane et/ou d'au moins un filtre UV du type dérivé de triazine pour augmenter le facteur de protection solaire.

## Patentansprüche

1. Zusammensetzung, die für den Schutz der Haut und/oder der Haare vor Ultraviolett-Strahlung vorgesehen ist, **dadurch gekennzeichnet, dass** sie in einem kosmetisch akzeptablen Träger mindestens enthält:
(a) ein Lichtschutzsystem, das imstande ist, UV-Strahlung zu filtern, das aus mindestens einem UV-Filter vom Typ der Filter, die von Benzylidencampher abgeleitet sind, und/ oder mindestens einem UV-Filter vom Typ der Filter, die von Dibenzoylmethan abgeleitet sind, und/oder mindestens einem UV-Filter vom Typ der Filter, die von Triazin abgeleitet sind, besteht;
(b) ein geradkettiges oder verzweigtes Dialkyltartrat, das vorzugsweise 1 bis 16 Kohlenstoffatome enthält;
wobei die Zusammensetzung kein 2,4,6-Tris[p-(2'-ethylhexyl-1'-oxycarbonyl)anilino]-1,3,5-triazin enthält.

2. Zusammensetzung nach Anspruch 1, wobei das Dialkyltartrat unter den C₁₋₁₆-Dialkyltartraten ausgewählt ist.

3. Zusammensetzung nach Anspruch 1 oder 2, wobei das Dialkyltartrat unter den C₁₋₄-Dialkyltartraten ausgewählt ist.

4. Zusammensetzung nach einem der Ansprüche 1 bis 3, wobei es sich bei dem Dialkyltartrat um Diethyltartrat handelt.

5. Zusammensetzung nach Anspruch 1 oder 2, wobei das Dialkyltartrat die folgende Struktur aufweist: 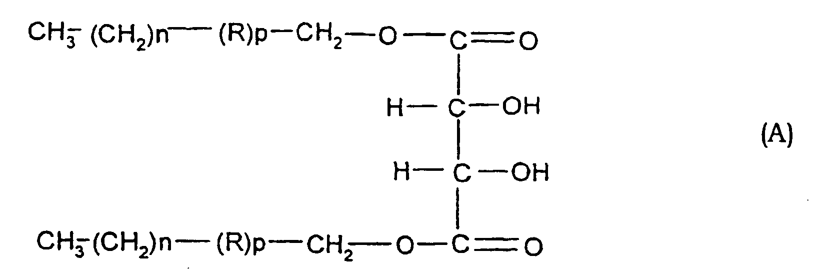 in der bedeuten:
R eine Gruppe der Formel: 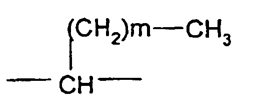
p die Zahl 0 oder 1;
n die Zahl 12 oder 13, wenn p gleich 0 ist, und
m + n die Zahl 8 oder 9 , wenn p gleich 1 ist.

6. Zusammensetzung nach einem der Ansprüche 1 bis 6, wobei das Dialkyltartrat in einer Konzentration verwendet wird, die im Bereich von 0,1 bis 10 Gew.-%, bezogen auf das Gesamtgewicht der Zusammensetzung, und vor allem im Bereich von 0,2 bis 5 Gew.-% liegt.

7. Zusammensetzung nach einem der Ansprüche 1 bis 6, wobei das Dibenzoylmethan-Derivat ausgewählt ist unter:
- 2-Methyldibenzoylmethan,
- 4-Methyldibenzoylmethan,
- 4-Isopropyldibenzoylmethan,
- 4-*tert*.-Butyldibenzoylmethan,
- 2,4-Dimethyldibenzoylmethan,
- 2,5-Dimethyldibenzoylmethan,
- 4,4'-Diisopropyldibenzoylmethan,
- 4-*tert*.-Butyl-4'-methoxydibenzoylmethan,
- 2-Methyl-5-isopropyl-4'-methoxydibenzoylmethan,
- 2-Methyl-5-*tert*.-butyl-4'-dibenzoylmethan,
- 2,4-Dimethyl-4'-methoxydibenzoylmethan,
- 2,6-Dimethyl-4-*tert*.-butyl-4'-methoxydibenzoylmethan.

8. Zusammensetzung nach einem der Ansprüche 1 bis 7, wobei es sich bei dem Dibenzoylmethan-Derivat um 4-*tert*.-Butyl-4'-methoxydibenzoylmethan handelt.

9. Zusammensetzung nach einem der Ansprüche 1 bis 7, wobei es sich bei dem Dibenzoylmethan-Derivat um 4-Isopropyldibenzoylmethan handelt.

10. Zusammensetzung nach einem der Ansprüche 1 bis 9, wobei das Benzylidencampher-Derivat ausgewählt ist unter:
- 3-Benzylidencampher,
- 4-Methylbenzylidencampher;
- 3-(4'-Trimethylammoniumbenzyliden)-camphermethylsulfat;
- Benzylidencamphersulfonsäure und ihren Salzen;
- 3-(3'-Sulfo-4'-methylbenzyliden)-campher;
- Polyacrylamidomethylbenzylidencampher.

11. Zusammensetzung nach einem der Ansprüche 1 bis 9, wobei das Benzylidenderivat ausgewählt ist unter:
- den Polydiorganosiloxanen mit Benzylidencampher-Gruppe;
- den Diorganosiloxanen mit kurzer, geradkettiger oder cyclischer Kette;
- den Triorganosilanen mit Benzylidencamphergruppe.

12. Zusammensetzung nach einem der Ansprüche 1 bis 9, wobei das Benzylidenderivat der folgenden allgemeinen Formel (III) 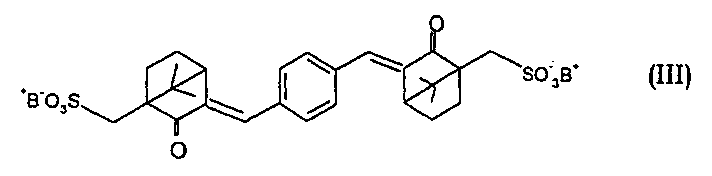 entspricht, in der B ein Wasserstoffatom, ein Alkalimetall, eine Gruppe NH(R)₃⁺, in der die Reste R, die gleich oder verschieden sein können, Wasserstoff, C₁₋₄-Alkyl- oder C₁₋₄-Hydroxyalkyl darstellen, oder eine Gruppe Mⁿ⁺/n bedeutet, wobei Mⁿ⁺ ein mehrwertiges Metallkation ist, worin n die Zahl 2 oder 3 oder 4 ist, wobei Mⁿ⁺ vorzugsweise ein Metallkation ist, das unter Ca²⁺, Zn²⁺, Mg²⁺, Ba²⁺, Al³⁺ und Zr⁴⁺ ausgewählt ist, einschließlich seiner Isomere.

13. Zusammensetzung nach einem der Ansprüche 1 bis 10, wobei es sich bei dem Benzylidencampher-Derivat um 4-Methylbenzylidencampher handelt.

14. Zusammensetzung nach einem der Ansprüche 1 bis 13, wobei das Triazin-Derivat der folgenden Formel (VI) 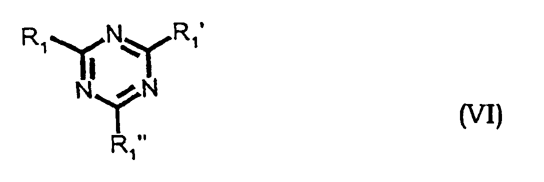 entspricht, in der R₁, R₁' und R₁", die gleich oder verschieden sein können, unter den einwertigen Resten der folgenden Formeln (1) und (2) ausgewählt sind: 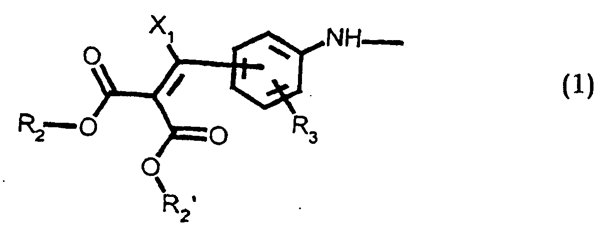 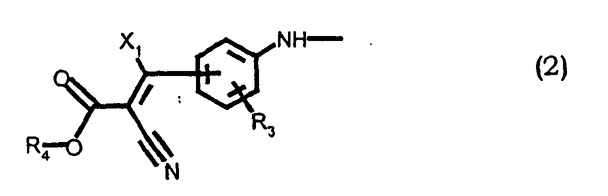 worin bedeuten:
- R₂ und R₂', die gleich oder verschieden sein können, einen geradkettigen oder verzweigten C₁₋₃-Alkylrest,
- R₃ ein Wasserstoffatom, einen geradkettigen oder verzweigten C₁₋₄-Alkylrest oder einen C₁₋₄-Alkoxyrest,
- R₄ einen geradkettigen oder verzweigten C₁₋₁₂-Alkylrest,
- X₁ Wasserstoff oder einen Phenylrest, der gegebenenfalls mit einem Halogenatom, C₁₋₄-Alkyl oder C₁₋₄-Alkoxy substituiert ist.

15. Zusammensetzung nach Anspruch 14, wobei das Triazin-Derivat ausgewählt ist unter:
- 2,4,6-Tris(4'-aminobenzalmalonsäurediethylester)-s-triazin,
- 2,4,6-Tris(4'-aminobenzalmalonsäurediisopropylester)-s-triazin,
- 2,4,6-Tris(4'-aminobenzalmalonsäuredimethylester)-s-triazin,
- 2,4,6-Tris(α-cyano-4-aminozimtsäureethylester)-s-triazin.

16. Zusammensetzung nach einem der Ansprüche 1 bis 15, wobei das Lichtschutzsystem zusätzlich ein oder mehrere weitere UV-Licht absorbierende organische Filter, die verschieden von den Benzylidencampher-Derivaten, Dibenzoylmethan-Derivaten und Triazin-Derivaten sind, ein oder mehrere anorganische Pigmente und/oder Nanopigmente oder Gemische dieser Substanzen enthält.

17. Zusammensetzung nach Anspruch 16, wobei die weiteren organischen UV-Filter, einzeln oder im Gemisch, unter 2-Phenylbenzimidazol-5-sulfonsäure und ihren Salzen, den Zimtsäure-Derivaten, den Salicylsäure-Derivaten, den Benzophenon-Derivaten, den β,β-Diphenylacrylat-Derivaten, den p-Aminobenzoesäure-Derivaten, Menthylanthranilat, den Polymerfiltern und den Siliconfiltern ausgewählt sind.

18. Zusammensetzung nach einem der Ansprüche 1 bis 17, wobei das Lichtschutzsystem in der Zusammensetzung in einer Konzentration enthalten ist, die im Bereich von 0,1 bis 30 Gew.-% und vorzugsweise 0,5 bis 15 Gew.-%, bezogen auf das Gesamtgewicht der Zusammensetzung, liegt.

19. Zusammensetzung nach einem der Ansprüche 1 bis 18, **dadurch gekennzeichnet, dass** das Lichtschutzsystem zusätzlich als ergänzende Lichtschutzmittel Pigmente oder Nanopigmente von Metalloxiden enthält, die gegebenenfalls umhüllt sind, die imstande sind, die UV-Strahlung physikalisch durch Streuung und/oder Reflexion zu blocken.

20. Zusammensetzung nach Anspruch 19, **dadurch gekennzeichnet, dass** die Pigmente oder Nanopigmente unter den Oxiden von Titan, Zink, Eisen, Zirconium, Cer und den Gemischen dieser Oxide, die gegebenenfalls umhüllt sind, ausgewählt sind.

21. Zusammensetzung nach einem der Ansprüche 1 bis 20, **dadurch gekennzeichnet, dass** sie außerdem mindestens ein Mittel zur Bräunung und/oder ein Mittel zur künstlichen Bräunung enthält.

22. Zusammensetzung nach einem der Ansprüche 1 bis 21, **dadurch gekennzeichnet, dass** sie in Form einer Dispersion mit kontinuierlicher wäßriger Phase vorliegt.

23. Zusammensetzung nach Anspruch 22, **dadurch gekennzeichnet, dass** sie in Form einer Gelcreme vorliegt.

24. Verwendung eines wie in einem der Ansprüche 1 bis 3 definierten Dialkyltartrats für die Herstellung einer Zusammensetzung, die für den Schutz der Haut und/oder der Haare vor Ultraviolett-Strahlung vorgesehen ist, die aus mindestens einem UV-Filter vom Typ der Filter, die von Benzylidencampher abgeleitet sind, und/oder mindestens einem UV-Filter vom Dibenzoylmethan-Typ und/oder mindestens einem W-Filter vom Triazin-Typ besteht, zur Erhöhung des Lichtschutzfaktors.

## Claims

1. Composition intended to protect the skin and/or the hair against ultraviolet radiation, **characterized in that** it comprises, in a cosmetically acceptable support, at least:
(a) a photoprotective system capable of screening out UV radiation, consisting of at least one UV screening agent of the type derived from benzylidenecamphor and/or at least one UV screening agent of the type derived from dibenzoylmethane and/or at least one UV screening agent of the type derived from triazine;
(b) a linear or branched dialkyl tartrate preferably having from 1 to 16 carbon atoms;
the said composition not containing 2,4,6-tris[p-(2'-ethylhexyl-1'-oxycarbonyl)anilino]-1,3,5-triazine.

2. Composition according to Claim 1, in which the dialkyl tartrate is chosen from C₁-C₁₆ dialkyl tartrates.

3. Composition according to Claim 1 or 2, in which the dialkyl tartrate is chosen from C₁-C₄ dialkyl tartrates.

4. Composition according to any one of Claims 1 to 3, in which the dialkyl tartrate is diethyl tartrate.

5. Composition according to Claim 1 or 2, in which the dialkyl tartrate corresponds to the following structure: 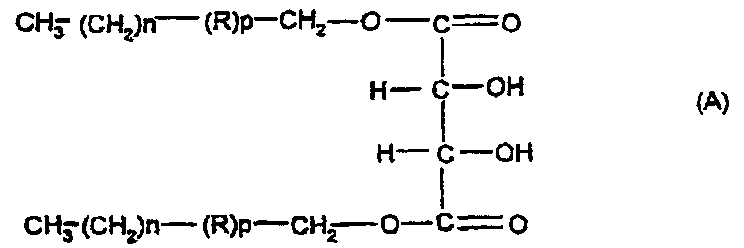 in which R denotes a group of formula: 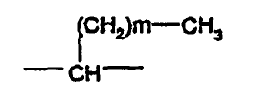 p is 0 or 1;
n is equal to 12 or 13 when p is equal to 0, and
m + n is equal to 8 or 9 when p is equal to 1.

6. Composition according to any one of Claims 1 to 5, in which the dialkyl tartrate is used at a concentration ranging from 0.1 to 10% by weight relative to the total weight of the composition, and more particularly from 0.2 to 5% by weight.

7. Composition according to any one of Claims 1 to 6, in which the dibenzoylmethane derivative is chosen from the group consisting of:
- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2,5-dimethyldibenzoylmethane,
- 4,4'-diisopropyldibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoylmethane,
- 2,6-dimethyl-4-tert-butyl-4'-methoxydibenzoylmethane.

8. Composition according to any one of Claims 1 to 7, in which the dibenzoylmethane derivative is 4-tert-butyl-4'-methoxydibenzoylmethane.

9. Composition according to any one of Claims 1 to 7, in which the dibenzoylmethane derivative is 4-isopropyldibenzoylmethane.

10. Composition according to any one of Claims 1 to 9, in which the benzylidenecamphor derivative is chosen from the group consisting of:
- 3-benzylidenecamphor;
- 4-methylbenzylidenecamphor;
- 3-(4'-trimethylammoniobenzylidene)camphor methyl sulphate;
- benzylidenecamphorsulphonic acid and its salts;
- 3-(3'-sulpho-4'-methylbenzylidene)camphor;
- polyacrylamidomethylbenzylidenecamphor.

11. Composition according to any one of Claims 1 to 9, in which the benzylidene derivative is chosen from the group consisting of:
- diorganopolysiloxanes containing a benzylidenecamphor function;
- diorganosiloxanes containing short, linear or cyclic chains;
- triorganosilanes containing a benzylidenecamphor function.

12. Composition according to any one of Claims 1 to 9, in which the benzylidene derivative corresponds to the general formula (III) below: 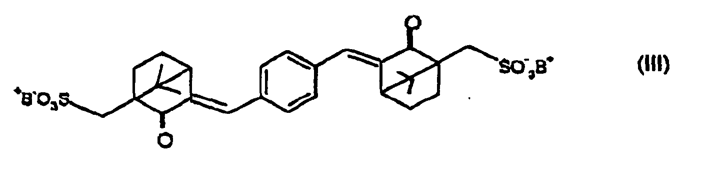 in which B denotes a hydrogen atom, an alkali metal or alternatively a radical NH(R)₃⁺ in which the radicals R, which can be identical or different, denote a hydrogen atom, a C₁-C₄ alkyl or hydroxyalkyl radical or alternatively a group Mⁿ⁺/n, Mⁿ⁺ denoting a polyvalent metal cation in which n is equal to 2 or 3 or 4, Mⁿ⁺ preferably denoting a metal cation chosen from Ca²⁺, Zn²⁺, Mg²⁺, Ba²⁺, Al³⁺ and Zr⁴⁺, as well as isomers thereof.

13. Composition according to any one of Claims 1 to 10, in which the benzylidenecamphor derivative is 4-methylbenzylidenecamphor.

14. Composition according to any one of Claims 1 to 13, in which the triazine derivative corresponds to formula (VI) below: 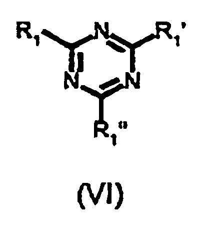 in which:
- R₁, R₁' and R₁", which may be identical or different, are chosen from the monovalent radicals of formula (1) or (2) below: 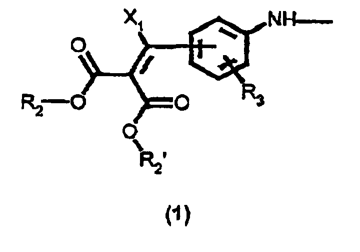 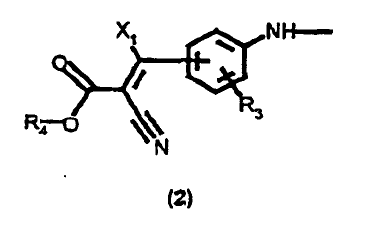
in which:
- R₂ and R₂', which may be identical or different, denote a linear or branched C₁-C₃ alkyl radical,
- R₃ denotes a hydrogen atom, a linear or branched C₁-C₄ alkyl radical or a C₁-C₄ alkoxy radical,
- R₄ denotes a linear or branched C₁-C₁₂ alkyl radical,
- X₁ represents a hydrogen atom or a phenyl radical optionally substituted with a halogen atom or with a C₁-C₄ alkyl radical or with a C₁-C₄ alkoxy radical.

15. Composition according to Claim 14, in which the triazine derivative is chosen from:
- 2,4,6-tris(diethyl 4'-aminobenzalmalonate)-s-triazine,
- 2,4,6-tris(diisopropyl 4'-aminobenzalmalonate)-s-triazine,
- 2,4,6-tris(dimethyl 4'-aminobenzalmalonate)-s-triazine,
- 2,4,6-tris(ethyl α-cyano-4-aminocinnamate)-s-triazine.

16. Composition according to any one of Claims 1 to 15, in which the photoprotective system also contains one or more additional organic UV-absorbing screening agents other than benzylidenecamphor derivatives, dibenzoylmethane derivatives or triazine derivatives, and one or more inorganic pigments and/or nanopigments, or mixtures of these.

17. Composition according to Claim 16, in which the additional organic UV screening agents are chosen, alone or as mixtures, from 2-phenyl-5-benzimidazolesulphonic acid and its salts, cinnamic derivatives, salicylic derivatives, benzophenone derivatives, β,β-diphenylacrylate derivatives, p-aminobenzoic acid derivatives, menthyl anthranilate, screening polymers and screening silicones.

18. Composition according to any one of Claims 1 to 17, in which the photoprotective system is present in the said composition at a concentration ranging from 0.1% to 30%, and preferably from 0.5 to 15%, by weight relative to the total weight of the composition.

19. Composition according to any one of Claims 1 to 18, **characterized in that** the photoprotective system also comprises, as complementary photoprotective agents, coated or uncoated metal oxide pigments or nanopigments capable of physically blocking out UV radiation by scattering and/or reflection.

20. Composition according to Claim 19, **characterized in that** the said pigments or nanopigments are chosen from titanium oxide, zinc oxide, iron oxide, zirconium oxide and cerium oxide, and mixtures thereof, which may be coated or uncoated:

21. Composition according to any one of Claims 1 to 20, **characterized in that** it also comprises at least one agent for artificially tanning and/or browning the skin.

22. Composition according to any one of Claims 1 to 21, **characterized in that** it is in the form of a dispersion containing an aqueous continuous phase.

23. Composition according to Claim 22, **characterized in that** it is in the form of a cream-gel.

24. Use of a dialkyl tartrate as defined in any one of Claims 1 to 3 for the manufacture of a composition intended to protect the skin and/or the hair against ultraviolet radiation, of the type comprising a photoprotective system capable of screening out UV radiation, this system consisting of at least one UV screening agent of the type derived from benzylidenecamphor and/or at least one UV screening agent of the dibenzoylmethane type, and/or at least one UV screening agent of the type derived from triazine, to increase the sun protection factor.
